Europäisches Patentamt

European Patent Office

Office européen des brevets

(11) Publication number: **0 398 522**
**A2**

# (12) EUROPEAN PATENT APPLICATION

(21) Application number: **90304444.4**

(22) Date of filing: **25.04.90**

(51) Int. Cl.5: **G01N 33/96, G01N 33/49,**
**G01N 1/30, G01N 15/14**

(30) Priority: **15.05.89 US 351901**

(43) Date of publication of application:
**22.11.90 Bulletin 90/47**

(84) Designated Contracting States:
**BE CH DE ES FR GB IT LI NL SE**

(71) Applicant: **TECHNICON INSTRUMENTS
CORPORATION**
**511 Benedict Avenue**
**Tarrytown New York 10591(US)**

(72) Inventor: **Kim, Young Ran**
**1-212 100G High Point Drive**
**Hartsdale, New York 10530(US)**
Inventor: **Martin, Grace E.**
**99 Park Drive**
**Mount Kisco, New York 10549(US)**
Inventor: **Paseltiner, Lynn**
**4 Roanoke Drive**
**Monoe, New York 10950(US)**
Inventor: **Benezra, Jeffrey**
**2460 Katonah Avenue, Apt. 8-A**
**Bronx, New York 10470(US)**

(74) Representative: **Wain, Christopher Paul et al**
**A.A. THORNTON & CO. Northumberland**
**House 303-306 High Holborn**
**London WC1V 7LE(GB)**

(54) **Optical standard for automated blood analysis.**

(57) A stable standard for electro-optical hematology measurements comprises an aqueous solution of
　　a) formaldehyde in a concentration of from about 0.5% to about 7.4% weight/volume;
　　b) a sugar or sugar alcohol in a concentration sufficient to adjust the refractive index of the optical standard to that of the sheath stream;
　　c) at least one buffer to maintain the pH of the solution between about 5.5 and 8.0; and
　　d) stained white blood cells in a concentration of from about 10,000 to about 35,000 cells per 40 $\mu$l.

EP 0 398 522 A2

## OPTICAL STANDARD FOR AUTOMATED BLOOD ANALYSIS

This invention relates to an optical standard for use with a blood analysis instrument, and more particularly to a standard composition comprising fixed, stained and stabilized white blood cells for setting the scatter and absorption gains in a hematology instrument such as the TECHNICON H*1 hematology analyzer (TECHNICON H*1 is a registered trademark of Technicon Instruments Corporation, Tarrytown, New York, U.S.A.)

There are five classes of normal white blood cells or leukocytes: neutrophils, lymphocytes, monocytes, eosinophils and basophils. It is a known medical diagnostic procedure to examine a dried, stained smear of blood on a microscope slide to determine the relative proportions of these five normal types of white blood cells, as well as the concentration of any abnormal cells. Such procedure is referred to as a differential white blood cell count and is described in Miale, J.B., "Laboratory Medicine -Hematology", pp. 822-830, 1126, 1127 and 1130, c.v. Mosby Company, St. Louis, MO (1967).

Recently, automated processes and automated flow system apparatus therefor have been developed to ease the burden of differential white blood cell counting, and examples of these are described in U.S. patent specifications nos. 3,741,875 and 4,099,917. They use cytochemical procedures to specifically identify and label individual cell types. Typically, a stream of stained and unstained cells flows through an optical view chamber where a photoelectric measuring process records the amplitudes of the light absorbed and, separately, the light scattered by each cell. Electronic signals are sorted by amplitude and classified into categories corresponding to the several cell types. Data are printed for the five major white cell types of whole blood. The specific chemical-identifying process stains the white blood cells. The light scattered roughly gauges the processed size of the cells. Because of the sophisticated measurement technology being developed, these instruments can be employed to follow the course of disease as well as to monitor the results of therapy more effectively.

An early procedure for preparing a cell suspension for use in such systems comprised treating an uncoagulated blood sample with a surfactant for about 1.5 minutes to precondition the red blood cells for lysis; thereafter adding a fixative to the cells for about one minute while maintaining a neutral pH; and incubating the mixture at 58°C to 60°C for about two minutes to lyse the red blood cells and fix the white blood cells, as described in U.S. patent specification no. 4,099,917.

A more recent method for the rapid determination of a differential white blood cell count in a sample is described in U.S. patent specification no. 4,801,549. This method comprises preparing a reagent solution of: (i) formaldehyde or paraformaldehyde; (ii) a surfactant; (iii) a sugar or sugar alcohol; and (iv) a buffer. This reagent solution is rapidly mixed with the sample to be analyzed to form a reaction mixture, wherein both the reagent solution and the sample are initially at a temperature of from about 20°C to about 28°C. Thereafter, the reagent mixture is heated to a temperature of from about 62°C to about 72°C within about 30 seconds in order to lyse the red blood cells and fix the white blood cells in the sample.

While existing methods provide the potential for more specific and accurate blood cell measurements, such specificity and accuracy are directly dependent upon the accuracy of the instrument employed in making the determinations. Since these cytochemical systems rely upon the processing of electro-optical signals, which are characteristic of the cells, to place various cell populations within appropriate ranges of signal size and/or staining intensity, any deviations due to variations in the gains in the signal processing circuitry within the instrument must be reduced to an insignificant minimum or substantially eliminated, otherwise reliably accurate results will not be obtained. It has been recognized that the instrument or, more appropriately, variations in the gain in signal processing circuitry, will affect the measured value. At present, a selection of normal samples is used and the gain of the instrument adjusted to place various cell populations within appropriate ranges of signal size. However, not only is it important to eliminate such system variation in the gain, but it is also desirable to standardize readings from one instrument to another so that different instruments will give the same results on the same blood sample. We have now devised a new standard for use in hematology and cytometry analysis, which is particularly useful in setting the absorption and scatter gains in the analysis system, and which has extended shelf life and is simple and inexpensive to prepare.

According to the present invention, there is provided a standard for use with an electro-optical hematology instrument utilizing the principles of sheath stream flow cytometry, the standard comprising an aqueous solution of:

a) formaldehyde in a concentration of from about 0.5% to about 7.4% weight/volume;

b) a sugar or sugar alcohol in a concentration sufficient to adjust the refractive index of the optical standard to that of the sheath stream;

c) at least one buffer to maintain the pH of the solution between about 5.5 and 8.0; and

d) stained white blood cells in a concentration of from about 10,000 to about 35,000 cells per 40 μl.

The standards of this invention are useful in lymphocyte subtyping methods practised on automated blood analyzers, for example, on a hematology or flow cytometry instrument. As used herein, the term hematology instrument includes any automated blood analyzer utilizing the principles of flow cytometry. While the invention will be described specifically with reference to the TECHNICON H*1 instrument it will be understood that the standards can be used with other hematology and cytometry analyzers and that the present invention is not limited to standards for the TECHICON H*1 instrument alone.

Generally, in accordance with the present invention, a fixed and stained white blood cell suspension is stabilized by use of an appropriate buffer, fixative and sugar in which the cells are sufficiently preserved with respect to scatter and absorption (staining intensity) properties so as to effectively allow proper adjustment of the scatter and absorption gains in a hematology instrument over an extended period of time. The standards of the invention are of a stable composition of fixed and stained white blood cells in a particular resuspending medium and are useful as scatter and absorption gain setting standards for a hematology instrument. The standards have excellent stability of optical properties over an extended period of time, for example, one year.

The invention also includes a method of setting the gain in the electro-optical system of a hematology instrument utilizing the principles of sheath stream flow cytometry, comprising:

a) passing through the flow channel of the flow cytometer a standard solution of the invention, to generate electro-optical signals representative of the population of a subset of white blood cells in said standard;

b) comparing the value of said signal from step a) with the known value of said standard; and

c) adjusting the gain of said hematology instrument to reflect the known value of said standard. In this method, the standard is passed through the flow channel of the cytometer to generate electro-optical signals which will permit the placement of various cell populations within appropriate ranges of signal size and/or staining intensity, for example, labelled lymphocytes (lymphocyte subtypes), unlabelled lymphocytes and stained neutrophils by enzymatic methods, e.g. peroxidase.

In the course of the following further description of the invention, reference will be made to the accompanying drawings, in which:

FIGURE 1 is a series of cytograms illustrating the stability of white blood cells (WBC's) suspended in a resuspending medium to provide a standard of the present invention compared with the stability of WBC's suspended in 30% propylene glycol;

FIGURE 2 is a graph demonstrating the stability of one embodiment of standard of the present invention over a 21-month period; and

FIGURE 3 is a graph demonstrating the stability of another embodiment of standard of the present invention over a period exceeding one year.

Formaldehyde or paraformaldehyde is used in the standards of this invention as a stabilizer of both the stained and unstained white blood cells. Preferably, formaldehyde is used and the preferred amount is from about 0.5% to about 7.4% weight/volume (w/v), most preferably from about 1.3% to about 3.6% weight/volume (w/v).

The sugar or sugar alcohol serves to raise the signal of the lymphocytes relative to platelets, red blood cell ghosts and other small particles, by matching the refractive index of the medium to that of the sheath of an appropriate flow cell. Suitable sugars or sugar alcohols include for example sucrose, fructose, dextrose, sorbital and mannitol.

The preferred sugar is dextrose, and the preferred sugar alcohol is sorbitol. Dextrose or sorbital should be used in an amount appropriate to match the refractive index of the cell suspension to that of the sheath. For example, we have prepared a resuspending medium using sorbitol in an amount of about 21% weight to volume with the resulting cell suspension having a refractive index of from about 1.364 to about 1.367. The concentration of sugar or sugar alcohol in the resuspending medium can be varied to match other desired refractive indices. When a sugar other than dextrose, or a sugar alcohol other than sorbitol, is used the amount should be adjusted so that the sugar or sugar alcohol is present in aproximately the same molar amount as the dextrose or sorbitol.

The buffer (or mixture of buffers) used should be suitable for maintaining the pH of the aqueous medium at about 5.5 to about 8, preferably at about 6.4 to about 6.6. Suitable buffers include sodium or potassium phosphates, 3-(N-morpholino) propane sulfonic acid (MOPS), N-2-acetamido-2-aminoethane sulfonic acid (ACES), and 4-(2-hydroxyethyl)-1-piperazine-ethanesulfonic acid (HEPES). A mixture of $Na_2HPO_4$ and $NaH_2PO_4$ is preferred. The mole ratio of $Na_2HPO_4$ to $NaH_2PO_4$ should preferably be from about 2.04:1 to about 0.81:1 to produce a series of solutions with a pH range of about 5.5 to about 8. The

3

concentration of such mixture in the resuspending medium is from about 0.075 molar (M) to about 1.125 M.

The resuspending medium i.e. the medium in which the WBC's are suspended to make a standard of the invention is an aqueous solution and, preferably, deionized water is used. The solution is prepared by combining the ingredients, in admixture, in water. A close watch should be maintained on the pH of the solution to ensure that it stays within the desired range. The solution may also include other additives as desired: for instance, ethylenediamine tetraacetic acid (EDTA) may be included as a metal chelator.

The following Table 1 presents a preferred composition for the resuspending medium, indicating the components in concentrations per liter of the resuspending medium:

TABLE 1

| Constituent | Quantity/Liter |
|---|---|
| $Na_2HPO_4$ | 4.6g |
| $Na_2H_2PO_4H_2O$ | 5.8g |
| Sorbitol | 210g |
| Formaldehyde (37%) | 41ml |
| Deionized Water | q.s. to 1000 ml |

The pH range of the composition is between about 6.4 and about 6.6 and the refractive index is between about 1.364 and about 1.367.

It will be appreciated that the concentration of white blood cells in the optical standard must fall between about 10,000 and about 35,000 cells per 40 $\mu$l (microliters); preferably, the concentration should be from about 15,000 to about 30,000 cells per 40 $\mu$l: and optimally, the concentration should be about 20,000 cells per 40 $\mu$l. If the concentration of white blood cells falls below about 10,000 cells per 40 $\mu$l, statistically satisfactory reproducibility cannot be obtained. At concentrations above 35,000 cells per 40 $\mu$l, significant coincidence in signals will tend to occur leading to inaccurate results.

In the following examples, various procedures for preparing optical standards in accordance with the present invention are described and experimental data are presented regarding the efficacy of such standards. Standard commercially available reagent grade materials were used whenever possible. It will be understood that the formulations and the procedures are illustrative only and that other ingredients, proportions and procedures can be employed.

Standard preparation from fixed white blood cells

Fixed white blood cells were obtained following established techniques recognized by those skilled in the art. White cells were first fixed by adding 125 ml of Tween/NaCl solution to 125 ml of the WBC fraction with gentle mixing continuously at room temperature for three minutes. ("Tween" is a trademark.) Thereafter, 250 ml of a fixative (6.5 g/l of sodium phosphate, dibasic; 4.0g/l of sodium phosphate, monobasic; 2.50 g/l of dextrose; 200ml/l of formaldehyde; 37% (w/w) of deionized water) were added to the solution and mixed in a water bath at approximately 100 oscillations per minute for six minutes. The mixture was then stored at room temperature. White cells were then washed by established procedures using sterile filtered water. The WBC count of this intermediate was thereafter determined using established techniques.

The quantity in milliliters (ml) of the WBC intermediate necessary to prepare one litre of the optical standard was determined in accordance with the following formula:

$$\frac{10,000 \text{ cells}/\mu l}{\text{WBC Count}/\mu l} \times 66.7 \text{ ml/l} = \text{ml of WBC intermediate required per litre optical standard}$$

The formulation quantity of the fixed WBC intermediate as determined above was added to 600 ml of a dye formulation and mixed for 10 minutes to stain the neutrophil population by their endogenous peroxidase activity. The dye formulation included 375 ml of deionized water, 425 ml phosphate buffer (250 mmol

4

sodium phosphate buffer or 500 mmol HEPES buffer and preservatives per litre of solution), 75 ml peroxidase dye (21.05 mmol 4-chloro-1-naphthol in 95% ethanol per litre of solution), and 7.5 ml peroxidase substrate (0.3% hydrogen peroxide and preservatives).

The fixed and stained WBC intermediate was then processed and finally added to the resuspending medium of Table 1 in accordance with the following procedure:

1. Aliquot the fixed and stained WBC intermediate into two 250 ml conical centrifuge tubes.

2. Centrifuge at 1000 g for three minutes (example: IEC Centra-7R with a rotating radius of 18.9 cm: Spin at 2200 RPM for three minutes).

3. The supernatant was carefully aspirated as close as possible to the cell button.

4. Vortex momentarily to loosen the cell button.

5. Replace supernatant with an equivalent amount of phosphate buffered saline with 0.2% bovine serum albumin and vortex to mix.

6. Repeat steps 2, 3 and 4.

7. Quantitatively transfer the fixed and stained WBC to a 1000 ml of the resuspending medium, and allow the suspension to mix for 15-30 minutes.

Standard preparation from whole blood

1. Collect fifteen 10 ml EDTA-anticoagulated fresh whole blood samples, and centrifuge for ten minutes. Collect buffy coat fraction and pool into one container. Mix well by inversion and obtain a white blood cell count by established protocols.

2. The quantity of pooled cells needed to yield $400 \times 10^6$ cells was determined using the following formula:

$$\frac{400 \times 10^6 \text{ cells}}{\text{WBC Count/ml}} = \text{volume (ml) of buffy coat fraction}$$

The calculated quantity of buffy coat fraction was added to each of two 250ml conical bottom centrifuge tubes labelled stained and unstained.

3. Add 100 ml of lysing reagent (ammonium chloride, 154.4 mmol; and suitable buffers) to each tube. Place on a rotator for 10 minutes. This causes lysis of the red blood cells.

4. Centrifuge both tubes for 10 minutes at 400g.

5. Aspirate the supernatant from both tubes and dislodge the cell pellet by using a vortex mixer.

6. Add 200 ml of cell washing solution (disodium EDTA, dihydrate, 3.09 mmol; tetrasodium EDTA, dihydrate, 3.82 mmol; albumin, 0.2%; and suitable buffers) to each tube and mix. Repeat steps 4 and 5.

7. Repeat step 6. The cells have now been washed two times.

8. Add 100 ml of lymphocyte fixative (formaldehyde, 7.4%, dextrose, 756.9 mmol and suitable buffers) to each tube. Place on a rotator for 10 minutes.

9. Repeat steps 4 and 5.

10. Repeat step 6 two times.

11. Add 200 ml of resuspending medium to the tube labelled "unstained". Set this tube aside. The remaining steps will only be performed on the tubed labelled "stained".

12. Add 134 ml of HEPES buffer (sodium hydroxide, 220 mmol; HEPES, 500 mmol; and preservatives) to the tube labelled "stained" and mix.

13. Add 24 ml of dye (4-chloro-1-naphthol, 21.05 mmol; and ethanol, 95%) to the tube labelled "stained" and mix.

14. Add 2.4 ml of substrate (hydrogen peroxide, 0.3%; and preservatives) to the tube labelled "stained" and place on a rotator for 10 minutes.

15. Repeat steps 4, 5 and 6.

16. Add 200 ml of resuspending medium to the tube labelled "stained" and mix.

A total cell count from both centrifuge tubes was obtained by using established techniques to verify that the total cell counts are within 4,000 cells of each other.

Both centrifuge tubes were emptied into a plain 1 litre beaker. ($V_1$)

To calculate the required volume of resuspending medium to obtain a total cell count of 20,000, the

5

following formula was used:

$$Cell\ Count = \frac{Stained\ Cell\ Count + Unstained\ Cell\ Count}{2}$$

$$V_1\ x\ \frac{cell\ count}{20,000} = V_2$$

$V_2$ = final volume (ml)

$V_2$ - V1 = volume (ml) of resuspending medium to be added

The volume of resuspending medium calculated was added to the beaker and the resulting solution mixed thoroughly.

Although the procedures of this invention are illustrated using automated equipment, it will be understood that it is also applicable to manual methods. The following Examples are given by way of illustration only. Data are presented using a standard TECHNICON H*1 analyzer.

## EXAMPLE 1

For the purpose of comparison, one portion of stained WBC was suspended in TECHNICON H*1 peroxidase sheath (30% propylene glycol) to match the refractive index of the sheath, whereas the other portion was suspended in the resuspending medium described above in Table 1. The data presented in Table 2 demonstrate the scatter and absorption stability of fixed WBC suspended in the resuspending medium, whereas the cells suspended in the peroxidase sheath are shown not to be stable. These data indicate one month stability. As used in this Example, and in subsequent Examples, the designation NEUT MEAN X AXIS refers to the mean absorption signals of the stained neutrophil population. The designation NEUT MEAN Y AXIS refers to the mean scatter signal of the stained neutrophils.

TABLE 2

| | STABILITY DATA OF OPTICAL STANDARD OF FIXED WBC IN | | | |
|---|---|---|---|---|
| | RESUSPENDING MEDIUM | | 30% PROPYLENE GLYCOL | |
| DAYS AT 4°C | NEUT MEAN X-AXIS | NEUT MEAN Y-AXIS | NEUT MEAN X-AXIS | NEUT MEAN Y-AXIS |
| 0 | 36.9 | 26.5 | 35.5 | 31.5 |
| 3 | 36.5 | 26.7 | 24.5 | 39.5 |
| 4 | 36.3 | 26.7 | (2) | (2) |
| 5 | 36.4 | 26.4 | | |
| 6 | 36.3 | 26.6 | | |
| 7 | 36.2 | 26.7 | | |
| 17 | 36.3 | 27.1 | | |
| 24 | 36.3 | 27.1 | | |
| 31 | 36.5 | 27.1 | | |
| 38 | 36.4 | 26.9 | | |
| NOTES: | | | | |

(1) Each point represents the mean of three (3) replicates.
(2) Cells became completely "destained" by Day 4 thereby causing too few cells to register in the NEUT and LPOS regions of the cytogram. These cells now appear in the LUC region of the cytogram.

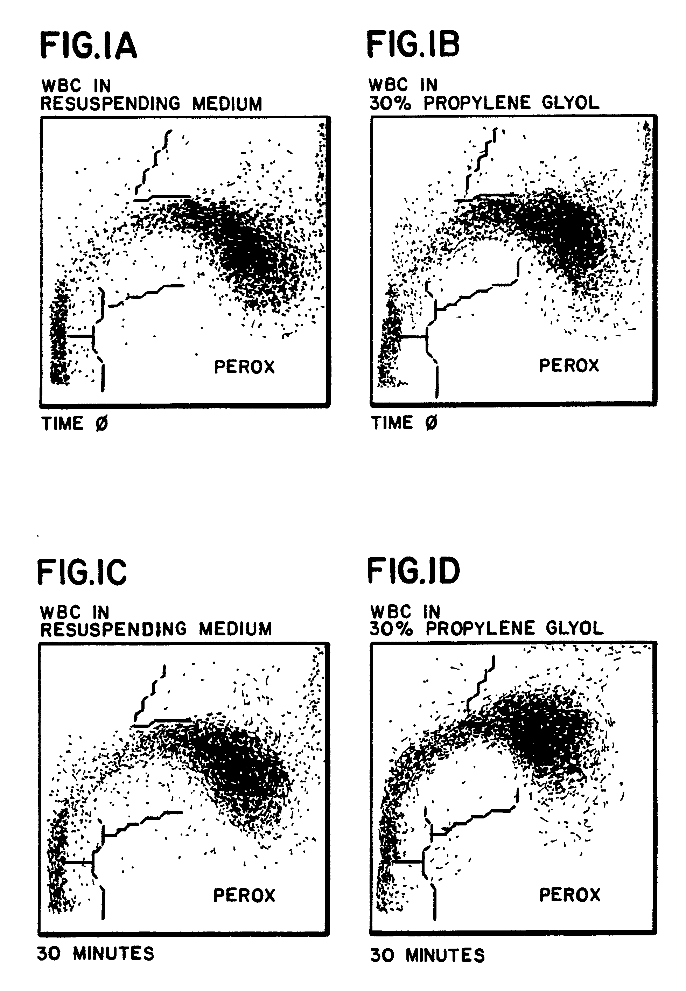

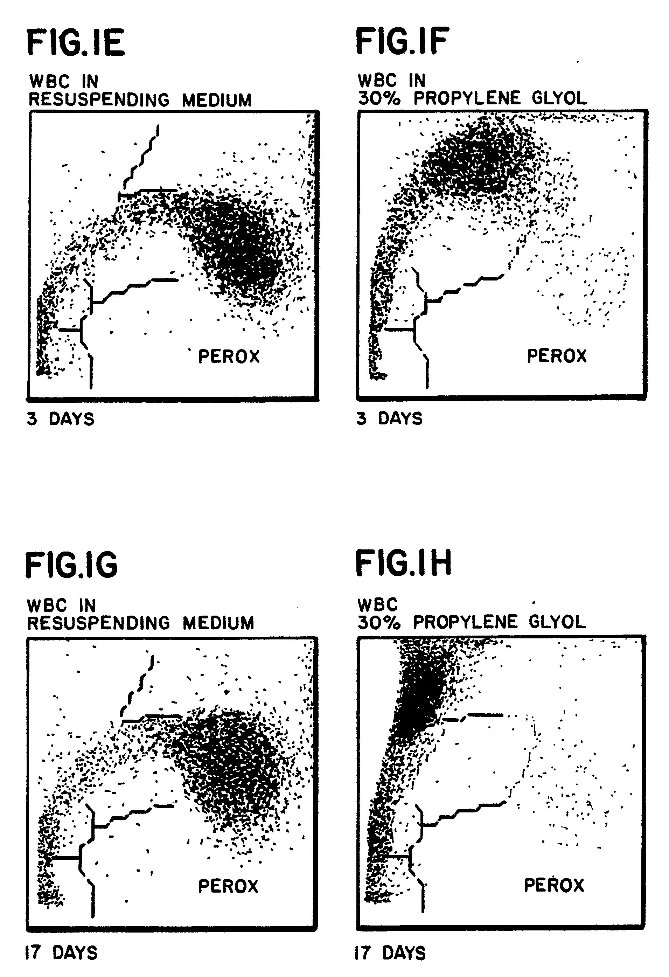

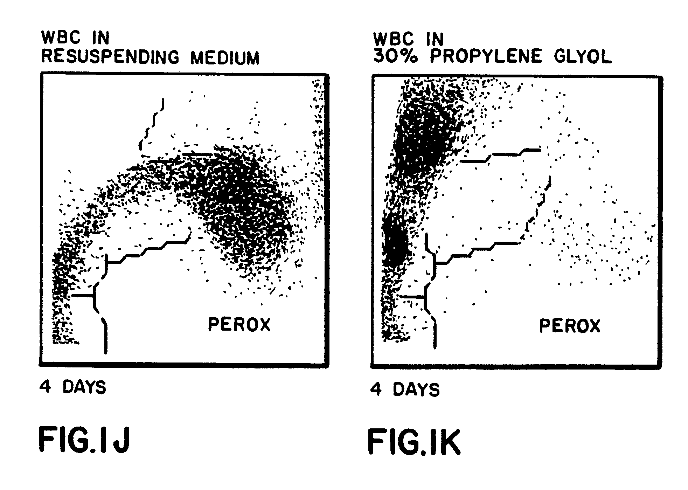

The cytogram of Fig. 1 illustrates this point further. In Fig. 1, WBCs suspended in the resuspending medium maintained the integrity of the cell signatures, whereas there is evidence of "destaining" within 30 minutes after resuspension of the cells in the 30% propylene glycol and by Day 4, destaining is complete. The 4-chloro-1-napthol dye is soluble in propylene glycol and this causes destaining of the neutrophils if solvents are not removed for an extended period of time.

EXAMPLE II

The formulation of the resuspending medium was varied by different percentages of formaldehyde; 0, 1, 2, 4, and 7.4. In each case, the sugar concentration was adjusted to maintain the proper refractive index. Fixed WBCs were stained, resuspended in each reagent and portions of each preparation were stored at 4°C and stressed at 37°C to derive Arrhenius extrapolation of the shelf life of the optical standard at 4°C. The results are presented in Table 3.

## TABLE 3

### EFFECT OF FORMALDEHYDE CONCENTRATION ON STABILITY OF STANDARD

| PERCENT FORMAL- DEHYDE | DAYS AT STRESS | 4°C | | 37°C | |
|---|---|---|---|---|---|
| | | NEUT MEAN X-AXIS | NEUT MEAN Y-AXIS | NEUT MEAN X-AXIS | NEUT MEAN Y-AXIS |
| 0 | 0 | 37.6 | 25.6 | 37.6 | 26.0 |
| | 1 | 37.0 | 25.1 | 35.9 | 26.3 |
| | 2 | 37.1 | 25.4 | 35.8 | 27.4 |
| | 3 | 37.0 | 25.5 | 34.4 | 26.1 |
| | 4 | – | – | 36.2 | 26.5 |
| | 7 | 37.1 | 25.9 | 32.3 | 20.8 |
| | 31 | 36.6 | 25.2 | QNS | QNS |
| 1 | 0 | 37.4 | 26.0 | 37.4 | 25.6 |
| | 1 | 37.0 | 25.6 | 36.3 | 26.4 |
| | 2 | 37.2 | 25.9 | 36.4 | 26.8 |
| | 3 | – | – | – | – |
| | 4 | – | – | 36.2 | 27.0 |
| | 7 | 37.1 | 26.4 | 36.1 | 27.7 |
| | 31 | 36.8 | 25.6 | 36.1 | 30.2 |
| 2 | 0 | 37.4 | 25.8 | 37.4 | 25.8 |
| | 1 | 37.0 | 25.6 | 36.3 | 26.5 |
| | 2 | 37.2 | 25.8 | 36.4 | 27.0 |
| | 3 | – | – | – | – |
| | 4 | – | – | 36.3 | 27.2 |
| | 7 | 37.1 | 26.3 | 36.2 | 27.8 |
| | 31 | 36.7 | 25.5 | 36.1 | 30.5 |

TABLE 3                                                          CONTINUED

| PERCENT FORMAL-DEHYDE | DAYS AT STRESS | 4°C | | 37°C | |
| --- | --- | --- | --- | --- | --- |
| | | NEUT MEAN X-AXIS | NEUT MEAN Y-AXIS | NEUT MEAN X-AXIS | NEUT MEAN Y-AXIS |
| 4 | 0 | 37.4 | 25.9 | 37.4 | 25.9 |
| | 1 | 37.0 | 25.8 | 36.3 | 26.7 |
| | 2 | 37.1 | 26.1 | 36.5 | 27.2 |
| | 3 | – | – | – | – |
| | 4 | – | – | 36.3 | 27.5 |
| | 7 | 37.1 | 26.6 | 36.2 | 28.3 |
| | 31 | 36.8 | 25.8 | 36.2 | 30.9 |
| 7.4 | 0 | 37.4 | 26.3 | 37.4 | 26.3 |
| | 1 | 37.0 | 26.1 | 36.4 | 27.0 |
| | 2 | 37.1 | 26.3 | 36.5 | 27.5 |
| | 3 | – | – | – | – |
| | 4 | – | – | 36.3 | 27.9 |
| | 7 | 37.1 | 26.8 | 36.3 | 28.8 |
| | 31 | 36.8 | 26.0 | 36.2 | 31.7 |

NOTE: (1) Each point represents the mean of three (3) replicates.

Samples stored at 4°C exhibit identical stability performance regardless of the presence or quantity of formaldehyde. Note that there was approximately 0.5 channel decrease in neutrophil staining (NEUT MEANx-AXIS) from Day 0 to Day 1 on all 4°C samples but staining remained unchanged thereafter. For samples stressed at 37°C, the 0% formaldehyde preparations exhibited a loss of scatter signal within 2 to 3 days. Therefore, adding formaldehyde enhanced the shelf life of the optical standard. Preparations containing from 1 to 7.4% formaldehyde maintained their scatter and absorption response and demonstrated equivalent stability performance. This would indicate that 1% formaldehyde is just as effective as higher concentrations.

## EXAMPLE III

Compositions similar to those of Example II, but having 0% and 1% formaldehyde concentrations, were tested and the sugar was changed from dextrose to sorbitol. The data are presented in Table 4.

TABLE 4

| | | 4°C | | | 37°C | | |
|---|---|---|---|---|---|---|---|
| PERCENT FORMAL- DEHYDE | DAYS AT STRESS | NEUT MEAN X-AXIS | NEUT MEAN Y-AXIS | TOTAL CELLS | NEUT MEAN X-AXIS | NEUT MEAN Y-AXIS | TOTAL CELLS |
| 0 | 0 | 37.1 | 24.7 | 23749 | 37.1 | 24.7 | 23749 |
| | 3 | 36.9 | 25.0 | 23732 | 35.3 | 27.0 | 18821 |
| | 4 | 37.2 | 25.2 | 23575 | 35.5 | 26.6 | 8480 |
| | 5 | 37.2 | 24.9 | 24145 | 37.9 | 28.7 | 2354 |
| | 17 | 37.2 | 25.2 | 23542 | – | – | – |
| | 45 | 37.2 | 25.3 | 23502 | – | – | – |
| 1 | 0 | 37.1 | 25.2 | 22307 | 37.1 | 25.2 | 22307 |
| | 3 | 36.9 | 25.5 | 22486 | 36.1 | 26.9 | 22180 |
| | 4 | 37.1 | 25.5 | 21630 | 36.1 | 27.1 | 22360 |
| | 5 | 37.1 | 25.5 | 22315 | 36.7 | 27.0 | 22915 |
| | 17 | 37.0 | 25.6 | 22440 | – | – | – |
| | 45 | 37.2 | 25.6 | 22134 | – | – | – |

NOTES: (1) Each point represents the mean of three (3) replicates.

These results reconfirm those observations noted in Table 3, i.e. at 4°C, both 0 and 1% formaldehyde preparations perform equivalently. When stressed at 37°C to derive the Arrhenius extrapolation of the shelf life of the standard at 4°C, staining intensity of the 0% formaldehyde preparation deteriorated with respect to scatter as evidenced by the significant loss of cells in the above Table.

EXAMPLE IV

Optical standards were prepared from both fixed white cells and fresh whole blood to evaluate relative stability.

A. Stability of optical standard prepared from fixed white blood cells.

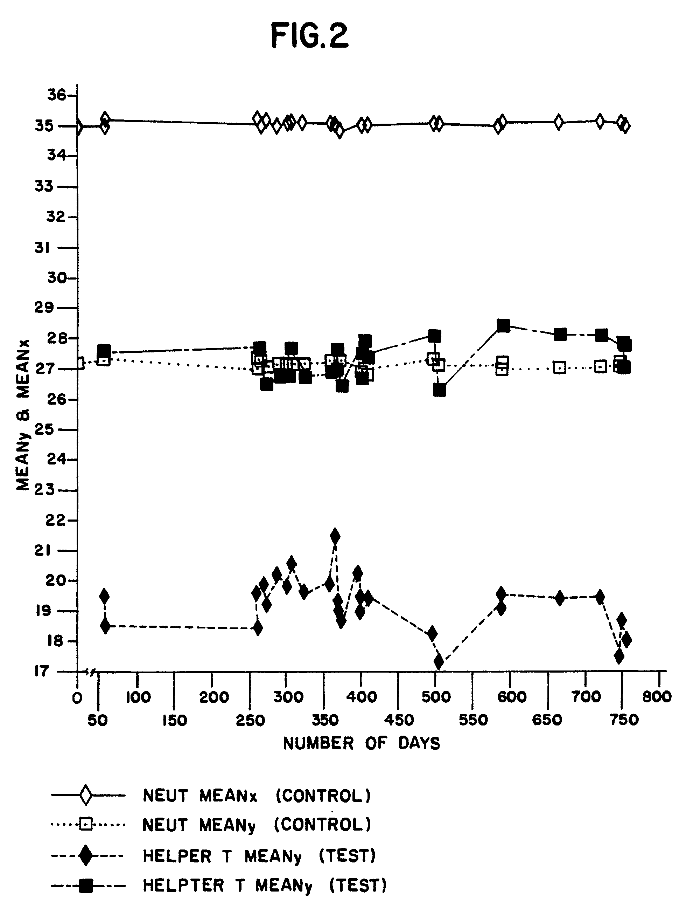

Following the recommended directions which are now public knowledge, whole blood was collected and processed according to the TECHNICON H*1 lymphocyte Helper T kit and used to verify the stability of the optical standard. The LYMPx and LYMPy gains of the TECHNICON H*1 instrument were set to the System Specific Value (SSV) of the optical standard and are designated as NEUT MEANx and NEUT MEANy in Fig. 2. The stained Helper T cells were then assayed at that gain setting. The L POS MEAN X (mean absorption signals from the labelled lymphocytes) and L POS MEAN Y (mean scatter) signals of labelled lymphocytes of the stained Helper T cells were recorded and plotted to monitor stability of the optical standard. When assaying the Helper T cells from normal blood donors, the L POS MEAN X is 18.5 ± 1.5 and the L POS MEAN Y is 27.7 ± 1.0. It will be appreciated by those skilled in the art that there may be a small number of values for normal cells which fall outside of the prescribed ranges. These are referred to as outliers, considered a statistical aberation and usually ignored. However, results which fall consistently outside this range would indicate an instability of the lot of optical standard used to set scatter and absorption gains since such constant outlining results would indicate that the gains were incorrectly set due to deterioration of the optical standard. As can be seen in Fig. 2, the values of L POS MEAN X and L POS MEAN Y fall within these ranges and stability has been shown for 24 months.

B. Stability of optical standard prepared with fresh whole blood.

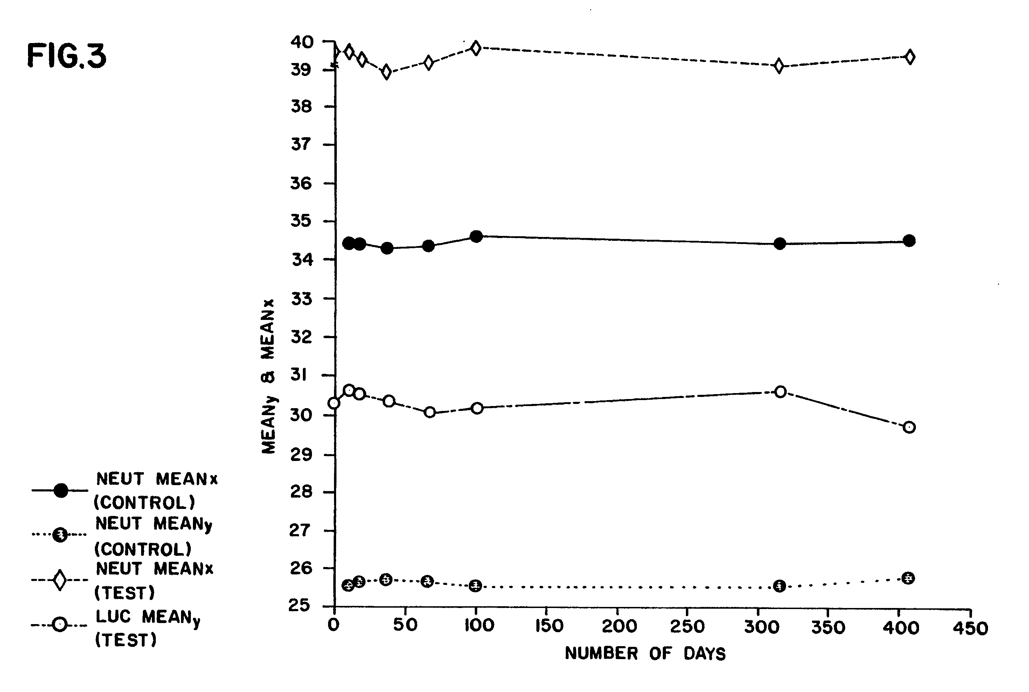

The LYMPx and LYMPy gains of the TECHNICON H*1 analyzer were set to the SSV of an in-date lot of

EP 0 398 522 A2

optical standard which had been proven to be stable by the method followed in A, and are designated NEUT MEANx (Control) and NEUT MEANy (Control) in Fig. 3. An optical standard made from fresh whole blood according to procedures described above was assayed at these gain settings and the NEUT MEANx and LUC MEANy (mean scatter signals of unstained neutrophil population) were recorded and plotted in Fig. 3 as NEUT MEANx (Test) and LUC MEANy (Test) respectively. The lack of any significant variation in the recorded values over time demonstrates the stability of the optical standard. Stability has been shown for over 12 months.

**Claims**

1. A standard for use with an electro-optical hematology instrument utilizing the principles of sheath stream flow cytometry, the standard comprising an aqueous solution of:
   a) formaldehyde in a concentration of from about 0.5% to about 7.4% weight/volume;
   b) a sugar or sugar alcohol in a concentration sufficient to adjust the refractive index of the optical standard to that of the sheath stream;
   c) at least one buffer to maintain the pH of the solution between about 5.5 and 8.0; and
   d) stained white blood cells in a concentration of from about 10,000 to about 35,000 cells per 40 $\mu$l.

2. A standard according to Claim 1, wherein the concentration of formaldehyde is from about 1.3% to about 3.6% weight/volume.

3. A standard according to Claim 1 or 2, wherein the sugar is sorbitol in an amount of about 22% weight/volume.

4. A standard according to Claim 1, 2 or 3, wherein the refractive index of the optical standard is from about 1.364 to about 1.367.

5. A standard according to any of Claims 1 to 4, wherein the pH is maintained at 6.4 to 6.6.

6. A standard according to any of Claims 1 to 4, wherein the buffer is sodium or potassium phosphate, 3-(N-morpholino) propane sulfonic acid, N-2-acetamido-2-aminoethane sulfonic acid, or 4-(2-hydroxy-ethyl)-1-piperazine-ethanesulfonic acid.

7. A standard according to Claim 6, wherein the buffer is a mixture of $Na_2HPO_4$ and $NaH_2PO_4$ in a mole ratio from about 2.04:1 to about 0.81:1.

8. A standard according to any of Claims 1 to 7, wherein the stained white blood cells are present at a concentration of from about 15,000 to about 30,000 cells per 40 $\mu$l, preferably approximately 20,000 cells per 40 $\mu$l.

9. A method of setting the gain in the electro-optical system of a hematology instrument utilizing the principles of sheath stream flow cytometry, comprising:
   a) passing through the flow channel of the flow cytometer a standard solution as claimed in any of claims 1 to 8, to generate electro-optical signals representative of the population of a subset of white blood cells in said standard;
   b) comparing the value of said signal from step a) with the known value of said standard; and
   c) adjusting the gain of said hematology instrument to reflect the known value of said standard.

10. The use of a standard solution as claimed in any of Claims 1 to 8 in the electro-optical hematology of blood.

Claims for the following Contracting State: ES

1. A method of setting the gain in the electro-optical system of a hematology instrument utilizing the principles of sheath stream flow cytometry, comprising:
   a) passing through the flow channel of the flow cytometer a standard solution to generate electro-optical signals representative of the population of a subset of white blood cells in said standard; wherein said standard solution comprises an aqueous solution of:
      i) formaldehyde in a concentration of from about 0.5% to about 7.4% weight/volume;
      ii) a sugar or sugar alcohol in a concentration sufficient to adjust the refractive index of the optical standard to that of the sheath stream;
      iii) at least one buffer to maintain the pH of the solution between about 5.5 and 8.0; and
      iv) stained white blood cells in a concentration of from about 10,000 to about 35,000 cells per 40 $\mu$l;
   b) comparing the value of said signal from step a) with the known value of said standard; and
   c) adjusting the gain of said hematology instrument to reflect the known value of said standard.

11

2. A method according to Claim 1, wherein the concentration of formaldehyde in the solution is from about 1.3% to about 3.6% weight/volume.

3. A method according to Claim 1 or 2, wherein in the the standard solution the sugar is sorbitol in an amount of about 22% weight/volume.

4. A method according to Claim 1,2 or 3, wherein the refractive index of the optical standard is from about 1.364 to about 1.367.

5. A method according to any of Claims 1 to 4, wherein the pH of the solution is maintained at 6.4 to 6.6.

6. A method according to any of Claims 1 to 4, wherein the buffer in the standard solution is sodium or potassium phosphate, 3-(N-morpholino) propane sulfonic acid, N-2-acetamido-2-aminoethane sulfonic acid, or 4-(2-hydroxyethyl)-1-piperazine-ethanesulfonic acid.

7. A method according to Claim 6, wherein the buffer is a mixture of $Na_2HPO_4$ and $NaH_2PO_4$ in a mole ratio from about 2.04:1 to about 0.81:1.

8. A method according to any of Claims 1 to 7, wherein the stained white blood cells are present in the standard solution at a concentration of from about 15,000 to about 30,000 cells per 40 $\mu$l, preferably approximately 20,000 cells per 40 $\mu$l.

9. A method of blood analysis utilising a hematology instrument, wherein the gain in the electro-optical system of the instrument is set by a method as claimed in any of Claims 1 to 8.

10. A hematology analyser instrument arranged to operate by sheath stream flow cytometry, which instrument has had its gain in the electro-optical system set by the method claimed in any of Claims 1 to 8.

EP 0 398 522 A2

**FIG.1A**

WBC IN
RESUSPENDING MEDIUM

PEROX

TIME Ø

**FIG.1B**

WBC IN
30% PROPYLENE GLYOL

PEROX

TIME Ø

**FIG.1C**

WBC IN
RESUSPENDING MEDIUM

PEROX

30 MINUTES

**FIG.1D**

WBC IN
30% PROPYLENE GLYOL

PEROX

30 MINUTES

## FIG.IE

WBC IN
RESUSPENDING MEDIUM

PEROX

3 DAYS

## FIG.IF

WBC IN
30% PROPYLENE GLYOL

PEROX

3 DAYS

## FIG.IG

WBC IN
RESUSPENDING MEDIUM

PEROX

I7 DAYS

## FIG.IH

WBC
30% PROPYLENE GLYOL

PEROX

I7 DAYS

WBC IN
RESUSPENDING MEDIUM

PEROX

4 DAYS

# FIG.IJ

WBC IN
30% PROPYLENE GLYOL

PEROX

4 DAYS

# FIG.IK

FIG.2

FIG.3

NUMBER OF DAYS

MEANy & MEANx

NEUT MEANx (CONTROL)
NEUT MEANy (CONTROL)
NEUT MEANx (TEST)
LUC MEANy (TEST)